# EUROPÄISCHE PATENTSCHRIFT

(11) **EP 0 727 967 B1**
(45) Veröffentlichungstag und Bekanntmachung des Hinweises auf die Patenterteilung: **10.02.1999**
(21) Anmeldenummer: 95900734.5
(22) Anmeldetag: 15.11.1994
(51) Int. Cl.: A61C 1/00, A61B 17/36

(54) **HANDSTÜCK, SOWIE VERFAHREN ZUR SPÜLUNG DES ARBEITSPUNKTES EINES AUS EINEM LICHTLEITER AUSTRETENDEN LASERLICHTSTRAHLS**
HAND-HELD UNIT AND A PROCESS FOR FLUSHING THE OPERATING POINT OF A LASER BEAM EMERGING FROM AN OPTICAL FIBER
PIECE A MAIN ET PROCEDE DE RINCAGE DU POINT DE FONCTIONNEMENT D'UN FAISCEAU LASER SORTANT D'UNE FIBRE OPTIQUE

(30) Priorität: 19.11.1993 DE 4339488
(43) Veröffentlichungstag der Anmeldung: 28.08.1996
(73) Patentinhaber: Rechmann, Peter, 40627 Düsseldorf-Unterbach (DE)
(72) Erfinder: Rechmann, Peter, 40627 Düsseldorf-Unterbach (DE)
(74) Vertreter: Dallmeyer, Georg, Dipl.-Ing.
(86) Internationale Anmeldenummer: EP9403776
(87) Internationale Veröffentlichungsnummer: WO9513759

(56) Entgegenhaltungen:
- EP-A- 0 247 746
- EP-A- 0 515 983
- PATENT ABSTRACTS OF JAPAN vol. 018, no. 131 (C-1175) 3. März 1994 & JP,A,05 317 329 (I N R KENKYUSHO) 3. Dezember 1993

## Beschreibung

Die Erfindung betrifft ein Handstück nach dem Oberbegriff des Anspruchs 1 sowie ein Verfahren nach Anspruch 8.

Derartige Handstücke werden für die Laserlichtführung bei medizintechnischen Geräten verwendet, wobei aufgrund der entstehenden Erwärmung im Arbeitspunkt des Laserlichtstrahls eine Spüleinrichtung vorhanden ist, die eine Flüssigkeit auf den Arbeitspunkt des Laserlichtstrahls oder in dessen Nähe richtet.

Als Handstücke sind Griffstücke zu verstehen, die die manuelle Laserlichtführung bei medizintechnischer Anwendung ermöglichen, wobei eine geradlinige oder auch abgewinkelte Lichtführung möglich ist.

Handstücke für die zahnärztliche Anwendung sind beispielsweise aus der EP-A-O 375 578, der DE-A-39 11 871 oder der EP-A-0 073 617 bekannt.

Nachteilig ist bei derartigen Handstücken mit Sprayspülung, daß die Spüleinrichtung die Wirksamkeit des Laserlichtstrahls im Arbeitspunkt beeinträchtigen kann, und daß die Kühlwirkung nicht ausreichend ist.

Aus der EP-A-0 515 983 ist ein Handstück mit einem Laserlichtleiter und einer Spüleinrichtung bekannt, die mit Hilfe einer Austrittsdüse einen laminaren Flüssigkeitsstrahl erzeugt, in den der Laserlichtstrahl koaxial eingekoppelt ist. Die Austrittsdüse ist am Ende einer Kammer angeordnet, die unter dem Flüssigkeitsdruck steht. Das Ende des Laserlichtleiters ist innerhalb der Kammer mit Abstand vor der Düse angeordnet, so daß aufgrund der Streuwirkung der Flüssigkeit in der Kammer und der sich erst in Strömungsrichtung hinter der Düse bildenden laminaren Strömung Einkopplungsverluste ergeben.

Der Erfindung liegt daher die Aufgabe zugrunde, ein Handstück derart weiterzubilden, daß bei einfachem Aufbau des Handstücks ein störungsfreier Betrieb möglich ist und bei dem der Laserlichtstrahl mit möglichst wenig Verlusten in den Flüssigkeitsstrahl eingekoppelt werden kann.

Zur Lösung dieser Aufgabe dienen die Merkmale des Anspruchs 1 bzw. 8.

Die Erfindung sieht vor, daß der Laserlichtstrahl in den von einem Gehäusekanal gebündelten und laminarisierten Flüssigkeitsstrahl der Spüleinrichtung durch Anordnen des freien Endes des Lichtleiters in dem Gehäusekanal koaxial eingekoppelt ist. Der Lichtleiter ist koaxial in dem Gehäusekanal für den Flüssigkeitsstrahl angeordnet. Der Gehäusekanal bündelt den Flüssigkeitsstrahl und umschließt dabei koaxial den Lichtleiter, so daß eine Parallelität des Laserlichtstrahls und des Flüssigkeitsstrahls gewährleistet ist.

Das Ende des Lichtleiters ist mit Abstand vor dem Ende des Gehäusekanals angeordnet. Auf diese Weise ist sichergestellt, daß der Laserlichtstrahl sich auf den Durchmesser des Flüssigkeitsstrahls erweitern kann.

Das Ende des Lichtleiters ist dabei mit einem solchen Abstand vor der Austrittsöffnung des Gehäusekanals angeordnet, daß der aus dem Lichtleiter austretende Laserlichtkegel an der Austrittsöffnung den Durchmesser des Flüssigkeitsstrahls erreicht. Die numerische Apertur des Lichtleiters bestimmt die Entfernung des freien Endes des Lichtleiters von der Austrittsöffnung, wobei der austretende Laserlichtstrahl sich möglichst genau im Bereich der Austrittsöffnung oder in Strömungsrichtung unmittelbar dahinter auf den Durchmesser des Flüssigkeitsstrahls erweitert haben soll. Der Laserlichtstrahl wird innerhalb des Flüssigkeitsstrahls an der Grenzschicht zwischen dem Flüssigkeitsstrahl und der umgebenden Luft total reflektiert, so daß der Flüssigkeitsstrahl als Lichtleiter dienen kann.

Die Einkopplung des Laserlichtstrahls in den Flüssigkeitsstrahl ermöglicht, die Totalreflektion des Laserlichtes an der Grenzfläche des Flüssigkeitsstrahls und somit den Flüssigkeitsstrahl als den Lichtleiter verlängerndes lichtleitendes Element derart zu verwenden, daß der Laserlichtstrahl exakt dem Verlauf des Flüssigkeitsstrahls folgt und der Arbeitspunkt des Laserlichtstrahls mit dem Auftreffpunkt des Flüssigkeitsstrahls übereinstimmt. Auf diese Weise wird erreicht, daß die Kühlung mit hoher Effektivität genau an der Stelle der Hitzeentwicklung, nämlich dem Arbeitspunkt des Laserlichtstrahls erfolgt.

Ein weiterer Vorteil des lichtleitenden Flüssigkeitsstrahls besteht darin, daß die abgetragene Substanz durch den Flüssigkeitsstrahl direkt fortgespült wird. Nicht von einem Flüssigkeitsstrom oder Luftstrom begleitete Laserlichtstrahlen haben nach dem Stand der Technik stets das Problem, daß der nach einigen Laserimpulsen erzeugte Krater in der bearbeiteten Substanz voller Abtrag ist, wobei der Laserlichtstrahl von dem bereits abgetragenen Material absorbiert wird und daher nur noch teilweise arbeitsfähig ist. Bei dem erfindungsgemäßen Flüssigkeitsstrahl mit integriertem Laserlichtstrahl wird aufgrund der ständig zugeführten Flüssigkeit die vom Laserlichtstrahl bearbeitete Stelle ständig gespült. Dies hat noch weitere Vorteile:

Das abgetragene Material ist erhitzt. Der sofortige Abtransport des heißen Abtrags aus dem Arbeitsbereich vermeidet eine zusätzliche unerwünschte Erwärmung der Grundsubstanz.

Die Laserlichtenergiedichte bleibt über einen längeren Flüssigkeitsstrahlabschnitt konstant. Auf diese Weise ist es möglich, das Handstück sowohl im Kontaktmode als auch im Abstand von dem Arbeitspunkt einzusetzen. Beim Kontaktmode wird die Austrittsöffnung direkt auf den Arbeitspunkt aufgesetzt. Während es nach dem Stand der Technik bei dem direkten Aufsetzen des Lichtleiters im Kontaktmode zu einem Abbrand des Lichtleiters kommt, kann es aufgrund der erfindungsgemäßen Einkopplung des Laserlichtstrahls in den Flüssigkeitsstrahl nicht zu einem Abbrand des Lichtleiters kommen.

Das Handstück kann direkt auf die zu bearbeitende Stelle aufgesetzt werden. Dies ermöglicht in vorteilhafter Weise mikrochirurgische Eingriffe bei Zähnen auch im Kontaktmodus. Auch in diesen Fällen ist eine ausreichende Kühlung der bearbeiteten Stelle sichergestellt. Die Anordnung des Lichtleiterendes innerhalb des Gehäusekanals, also vor der Austrittsöffnung schützt das Lichtleiterende vollständig vor Abbrand und mechanischer Zerstörung. Bei der Ablation von Zahnhartsubstanz bilden sich extrem harte Mikropartikel, die durch den Ablationsdruck auf die Faser zurückgesprengt werden und das Ende des Lichtleiters beschädigen können. Dies hat auch zur Folge, daß die Energieverteilung im austretenden Laserlichtstrahl inhomogen werden kann. Dadurch, daß gemäß der Erfindung das Ende des Lichtleiters geschützt innerhalb des Gehäusekanals angeordnet ist, kann ein unerwünschter Abbrand des Faserendes nicht entstehen.

Da das Ende des Lichtleiters mit Abstand vor der Austrittsöffnung des rohrförmigen Teils angeordnet ist, ist stets die Kühlung der bearbeiteten Oberfläche gesichert, da der Lichtleiter nicht direkt auf der zu bearbeitenden Oberfläche aufliegen kann.

Als Spülflüssigkeit ist auch eine Flüssigkeit einsetzbar, deren Brechungsindex an den Brechungsindex der zu bearbeitenden Oberfläche angepaßt ist. Auf diese Weise kann die Effektivität des Laserlichtstrahls aufgrund der verringerten Reflektion an der zu bearbeitenden Oberfläche erhöht werden.

Weitere vorteilhafte Merkmale der Erfindung sind den Unteransprüchen zu entnehmen.

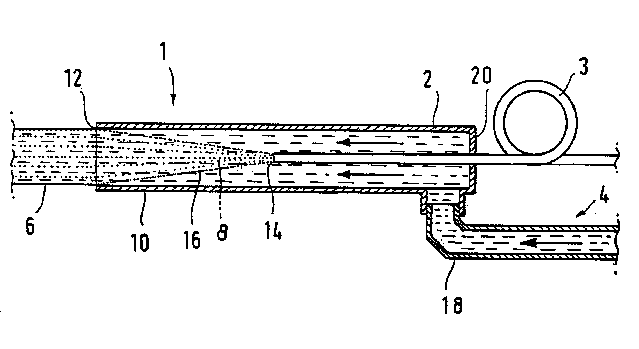

Im folgenden wird unter Bezugnahme auf die Zeichnung ein Ausführungsbeispiel der Erfindung näher erläutert:

Die einzige Figur zeigt eine nicht maßstabsgerechte Funktionsprinzipskizze der Erfindung.

Das Handstück 1 weist ein Gehäuse 2 auf, das in der schematischen Darstellung des Funktionsprinzips zugleich einen Gehäusekanal 10 zur Bündelung eines Flüssigkeitsstrahls 6 darstellt. Der Flüssigkeitsstrahl 6 wird von einer im einzelnen nicht dargestellten Spüleinrichtung 4 über eine Anschlußleitung 18 des Gehäusekanals 10 an dem der Austrittsöffnung 12 gegenüberliegenden Ende zugeführt. Koaxial zu dem Gehäusekanal 10 und gegenüber dem Gehäuse 2 abgedichtet ist auf der der Austrittsöffnung 12 gegenüberliegenden Stirnseite 20 des Gehäuses ein Lichtleiter, z.B. eine lichtleitende Faser 3, eingeführt, über die ein Laserlichtstrahl 8 in den mit Flüssigkeit gefüllten Gehäusekanal 10 eingeleitet wird.

Die lichtleitende Faser 3 endet mit Abstand vor der Austrittsöffnung 12 in dem Kanal, wobei das Ende 14 der lichtleitenden Faser 3 mit einem solchen Abstand vor der Austrittsöffnung 12 des Gehäusekanals 10 angeordnet ist, daß der aus der lichtleitenden Faser 3 austretende Laserlichtkegel 16 an der Austrittsöffnung 12 oder in Strömungsrichtung unmittelbar hinter der Austrittsöffnung 12 den Durchmesser des Flüssigkeitsstrahls 6 erreicht. Die numerische Apertur der lichtleitenden Faser 3 (das ist der Sinus des halben Austrittswinkels des Laserlichtstrahls 8) bestimmt den Abstand des Endes 14 der Faser 3 von der Austrittsöffnung 12. Der austretende Laserlichtstrahl 8 soll sich möglichst genau im Bereich der Austrittsöffnung 12 auf den Durchmesser des Flüssigkeitsstrahls 6 aufgeweitet haben. Alternativ kann auch der Laserlichtkegel 16 sich erst in Strömungsrichtung kurz hinter der Austrittsöffnung 12 auf den Durchmesser des Flüssigkeitsstrahls 6 erweitern, damit keine Absorption an der inneren Oberfläche des Gehäusekanals stattfinden kann. Der Laserlichtkegel 16 wird an dem Übergang Flüssigkeit-Luft total reflektiert, so daß der Flüssigkeitsstrahl 6 als Verlängerung der lichtleitenden Faser 3 dient.

Der auf den Durchmesser des Flüssigkeitsstrahls 6 erweiterte Laserlichtstrahl 8 behält über einen längeren Streckenabschnitt des Flüssigkeitsstrahls 6 seine Energiedichte, so daß das Handstück 1 sowohl in Kontaktbetriebsweise als auch mit Abstand von der zu bearbeitenden Substanz verwendet werden kann. Dieser Abstand kann bis zu 50 mm betragen und ggf. mit Hilfe eines Abstandhalters auf einen vorbestimmten Abstand eingestellt werden. Der typische Flüssigkeitsstrahldurchmesser beträgt zwischen 0,5 und 2,5 mm, vorzugsweise 1,5 mm.

Das Ende 14 der Faser 3 sollte nicht zu weit vor der Austrittsöffnung 12 enden, da es dann zu Reflektionen des Laserlichtstrahls an der Innenfläche des Gehäusekanals 10 mit teilweiser Absorption der Laserlichtenergie kommen kann. Steht das Ende 14 der Faser 3 in Relation zur Austrittsöffnung 12 zu weit vor, so ist dies zumindest für den Kontaktbetrieb des Handstücks 1 nachteilig, da die Laserlichtenergiedichte an der Austrittsöffnung 12 höher ist, d.h. innerhalb eines Streckenabschnitts direkt hinter der Austrittsöffnung 12 sind unterschiedliche Laserlichtintensitäten vorhanden, da der Lichtkegel sich erst mit Abstand von der Austrittsöffnung auf den Flüssigkeitsstrahldurchmesser erweitert hat.

Es können unterschiedliche Lichtleiter 3 eingesetzt werden. So können beispielsweise lichtleitende Fasern mit unterschiedlichem Durchmesser oder mit unterschiedlicher numerischer Apertur bei gleichem Faserdurchmesser eingesetzt werden.

Die Spülflüssigkeit für den Flüssigkeitsstrahl 6 wird über die Anschlußleitung 18 so in den Gehäusekanal 10 eingeleitet, daß in dem Gehäusekannal eine laminare Flüssigkeitsströmung entsteht. Hierzu kann es zweckmäßig sein, die Flüssigkeit an anderer Stelle in den Gehäusekanal 10 einzuleiten als in der Prinzipskizze dargestellt. Die Spülflüssigkeit kann mit leichtem Überdruck zugeführt werden. Eine zweckmäßige Flüssigkeitsmenge bei zahnärztlicher Anwendung des Handstücks beträgt ca. 30 bis 100 ml/min.

So könnte es abweichend von der Prinzipskizze in der einzigen Fig. sinnvoll sein, die Spülflüssigkeit parallel zum austretenden Flüssigkeitsstrahl 6 einzuleiten, während die biegsame lichtleitende Faser 3 bogenförmig in die koaxiale Position in den Gehäusekanal 10 überführt wird ohne durch die Stirnseite 20 hindurchgeführt zu werden.

Zwischen der Anschlußleitung 18 und dem Gehäusekanal 10 können auch Mittel zur Vergleichmäßigung der Flüssigkeitsströmung zwischengeschaltet sein, um ein möglichst ideales laminares Strömungsprofil zu erreichen. Des weiteren sind Maßnahmen zur Reibungsreduzierung an den Innenflächen des Gehäusekanals möglich, um die Ausbildung eines laminaren Strömungsprofils zu begünstigen.

Als Spülflüssigkeit kann Wasser oder auch eine Flüssigkeit verwendet werden, deren Brechungsindex an den Brechungsindex der zu bearbeitenden Oberfläche angepaßt ist. Dies führt zu geringeren Reflektionen an der zu bearbeitenden Oberfläche, z.B. Zahnoberfläche, da die Reflektion proportional zum Unterschied der Brechungsindices ist. Dabei ist zu berücksichtigen, daß die Verwendung einer Spülflüssigkeit mit einem anderen Brechungsindex den Aufweitungswinkel des Laserlichtkegels 16 beeinflußt.

Der Brechungsindex der Zahnoberfläche (Hydroxylapatit) beträgt n = 1,63. Bei Verwendung von Wasser (Brechungsindex n = 1,34) erhält man bereits eine gute Annäherung an den Brechungsindex einer Zahnoberfläche. Durch Zugabe von Kalziumchlorid zu der Spülflüssigkeit läßt sich der Brechungsindex auf n = 1,52 verbessern.

Der Spülflüssigkeit kann auch ein Fluorid (z.B. CaF₂) hinzugefügt werden. Auf diese Weise würde die bearbeitete Zahnfläche automatisch fluoridiert werden. Es hat sich gezeigt, daß aufgrund der gleichzeitigen Anwendung von Fluoriden und des Laserlichtstrahls die Effektivität der Fluoridierung verbessert wird und die bearbeiteten Zahnflächen deutlich weniger kariesanfällig werden. Dies ist voraussichtlich darauf zurückzuführen, daß Laserlicht die chemische Reaktion beschleunigt.

Ein flüssigkeitsgeführter Laserlichtstrahl ist auch besonders vorteilhaft zur Desensibilisierung von Zahnhälsen anwendbar.

Die Laserlichtleitung mit dem Flüssigkeitsstrahl 6 ist dann besonders effektiv, wenn der Laserlichtstrahl 8 nicht oder nur minimal von der Spülflüssigkeit absorbiert wird. Beste Ergebnisse sind bei Spülflüssigkeiten auf Wasserbasis mit Laserlicht einer Wellenlänge zwischen 300 und 700 nm, vorzugsweise zwischen 350 und 520 nm erzielbar.

Für andere Wellenlängenbereiche können andere Spülflüssigkeiten einen besseren Wirkungsgrad erreichen, z.B. im UV-Wellenlängenbereich organische Flüssigkeit wie Alkane, im IR-Wellenlängenbereich wasserfreie anorganische Flüssigkeiten, wie perhalogenierte Kohlenstoffverbindungen.

## Patentansprüche

1. Handstück (1) für die zahnärztliche Behandlung mit Laserlichtstrahl (8), mit einem Gehäuse (2), mit einem in dem Gehäuse (2) gehaltenen Laser-Lichtleiter (3), und mit einer Spüleinrichtung (4), die einen Flüssigkeitsstrahl (6) in den Bereich des Arbeitspunktes des Laserlichtstrahls (8) richtet, wobei das Gehäuse (2) den Lichtleiter (3) koaxial umschließt, und wobei der Laserlichtstrahl (8) in den Flüssigkeitsstrahl (6) koaxial eingekoppelt ist, so daß der Flüssigkeitsstrahl als Lichtleiter verwendbar ist,
**dadurch gekennzeichnet**, daß das Gehäuse (2) einen Kanal (10) aufweist, in dem die Flüssigkeit bis zum Austrittsende des Kanals (10) zu einem laminaren Flüssigkeitsstrahl (6) gebündelt wird, und daß das Ende des Lichtleiters (3) innerhalb des Gehäusekanals (10) mit Abstand vor dem Austrittsende (12) des Gehäusekanals (10) in dem laminaren Flüssigkeitsstrahl (6) angeordnet ist.

2. Handstück nach Anspruch 1, dadurch gekennzeichnet, daß das Ende (14) des Lichtleiters (3) mit einem solchen Abstand vor dem Ende des Gehäusekanals (10) angeordnet ist, daß der aus dem Lichtleiter (3) austretende Laserlichtkegel (16) an dem Ende oder unmittelbar hinter dem Ende (12) des Gehäusekanals (10) den Durchmesser des Flüssigkeitsstrahls (6) erreicht.

3. Handstück nach einem der Ansprüche 1 oder 2, dadurch gekennzeichnet, daß die zugeführte Flüssigkeitsmenge ca. 30 bis 100 ml/min beträgt.

4. Handstück nach einem der Ansprüche 1 bis 3, dadurch gekennzeichnet, daß der Strahldurchmesser des aus dedem Ende des Gehäusekanals (10) heraustretenden Flüssigkeitsstrahls (6) mit eingekoppeltem Laserstrahl (8) ca. 0,5 bis 2,5 mm, vorzugsweise ca. 1,5 mm, beträgt.

5. Handstück nach einem der Ansprüche 1 bis 4, dadurch gekennzeichnet, daß die Flüssigkeit im wesentlichen aus Wasser besteht.

6. Handstück nach einem der Ansprüche 1 bis 5, dadurch gekennzeichnet, daß das Laserlicht eine Wellenlänge von 300 bis 700 nm, vorzugsweise ca. 350 bis 520 nm aufweist.

7. Handstück nach einem der Ansprüche 1 bis 6, dadurch gekennzeichnet, daß die Spülflüssigkeit einen Brechungsindex aufweist, der dem Brechungsindex der zu bearbeitenden Oberfläche angepaßt ist.

8. Verfahren zur Spülung des Arbeitspunktes eines aus einem Lichtleiter (3) austretenden Laserlichtstrahls (8), bei dem eine Spülflüssigkeit zu einem Flüssigkeitsstrahl (6) gebündelt wird, der Laserlichtstrahl (8) in den gebündelten Flüssigkeitsstrahl (6) eingekoppelt wird, wobei der Flüssigkeitsstrahl (6) als Lichtleiter (3) und zugleich als Kühlmittel für den Arbeitspunkt des Laserlichtstrahls (8) verwendet wird, und ein laminarer Flüssigkeitsstrahl (6) in einem Gehäusekanal (10) gebildet wird, gekennzeichnet durch
- das koaxiale Einkoppeln des Laserlichtstrahls (8) in den laminaren Flüssigkeitsstrahl (6) innerhalb des Gehäusekanals (10) mit Abstand vor dem Austrittsende (12) des Gehäusekanals (10).

9. Verfahren nach Anspruch 8, dadurch gekennzeichnet, daß der Lichtkegel des aus dem Lichtleiter (3) austretenden Laserlichtes durch Wählen eines geeigneten Durchmessers des Lichtleiters (3) bzw. des Gehäusekanals (10) oder durch entsprechendes Positionieren des Lichtleiters (14) innerhalb des Gehäusekanals (10) so eingestellt wird, daß der Durchmesser des Lichtkegels (16) am Ende (12) des Gehäusekanals (10) den Durchmesser des Flüssigkeitsstrahls (6) erreicht.

10. Verfahren nach einem der Ansprüche 8 oder 9, dadurch gekennzeichnet, daß der Gehäusekanal (10) im Kontaktmode auf den Arbeitspunkt aufgesetzt wird.

11. Verfahren nach einem der Ansprüche 8 oder 9, dadurch gekennzeichnet, daß der Gehäusekanal (10) mit vorbestimmtem Abstand von dem Arbeitspunkt gehalten wird.

12. Verfahren nach einem der Ansprüche 8 bis 11, gekennzeichnet durch die Verwendung einer Spülflüssigkeit, deren Brechungsindex an den Brechungsindex der zu bearbeitenden Oberfläche angepaßt ist.

13. Verfahren nach einem der Ansprüche 8 bis 12, gekennzeichnet durch die Verwendung von Wasser mit einem Calziumchloridzusatz als Spülflüssigkeit.

14. Verfahren nach einem der Ansprüche 8 bis 12, gekennzeichnet durch die Verwendung eines Desinfektionsmittels als Zusatz zur Spülflüssigkeit.

15. Verfahren nach einem der Ansprüche 8 bis 12, gekennzeichnet durch die Verwendung von Fluoriden als Zusatzmittel zur Spülflüssigkeit.

## Claims

1. A hand-held unit (1) with a housing (2), with a laser light conductor (3) held in the housing (2), particularly for the dental treatment with a laser light beam (8), and with a flushing means (4) directing a liquid jet into the region of the operating point of the laser light beam (8), the housing (2) coaxially enclosing the light conductor (3), and wherein the laser light beam (8) is coaxially coupled into the liquid jet (6) so that the liquid jet may be used as a light conductor,
**characterized in**
that the housing (2) comprises a channel (10) in which the liquid is bundled into a laminar liquid jet (6) before reaching the channel outlet, and that the end of the light conductor (3) is arranged within the housing channel (10) and within the liquid jet (6) at a distance before the outlet (12) of the housing channel (10).

2. The hand-held unit according to claim 1, characterized in that the end (14) of the light conductor (3) is arranged at such a distance before the outlet of the housing channel (10) that the laser light cone (16) emerging from the light conductor (3) reaches or substantially reaches the diameter of the liquid jet (6) at the outlet (12) or directly behind the outlet (12) of the housing channel (10).

3. The hand-held unit according to one of claims 1 or 2, characterized in that the supplied liquid quantity amounts to from about 30 to 100 ml/min.

4. The hand-held unit according to one of claims 1 to 3, characterized in that the jet diameter of the liquid jet (6) emerging from the outlet of the housing channel (10), with the laser beam (8) coupled therein, amounts to from about 0.5 to 2.5 mm, preferably to about 1.5 mm.

5. The hand-held unit according to one of claims 1 to 4, characterized in that the liquid substantially consists of water.

6. The hand-held unit according to one of claims 1 to 5, characterized in that the laser light has a wavelength from 300 to 700 nm, preferably from about 350 to 520 nm.

7. The hand-held unit according to one of claims 1 to 6, characterized in that the flushing liquid has a refractive index adapted to that of the surface to be worked upon.

8. A process for flushing the operating point of a laser light beam (8) emerging from a light conductor (3), wherein a flushing liquid is bundled to form a liquid jet (6), the laser light beam (8) is coupled into the bundled liquid jet (6), the liquid jet (6) being used as a light conductor (3) and as a cooling agent for the operating point of the laser light beam (8) at the same time, and a laminar liquid jet (6) is formed in a housing channel (10),
characterized by
coaxially coupling the laser light beam (8) into the liquid jet (6) within the housing channel (10) at a distance before the outlet (12) of the housing channel (10). .

9. The process according to claim 8, characterized in that the light cone of the laser light emerging from the light conductor (3) is set by selecting a suitable light conductor (3) diameter or housing channel (10) diameter or by accordingly positioning the light conductor (14) within the housing channel (10) such that the diameter of the light cone (16) reaches the diameter of the liquid jet (6) at the outlet end (12) of the housing channel (10).

10. The process according to one of claims 8 or 9, characterized in that the housing channel (10) is set upon the operating point in the contact mode.

11. The process according to one of claims 8 or 9, characterized in that the housing channel (10) is held at a predetermined distance from the operating point.

12. The process according to one of claims 8 to 11, characterized by the use of a flushing liquid whose refractive index is adapted to the refractive index of the surface to be worked.

13. The process according to one of claims 8 to 12, characterized by the use of water with a calcium chloride addition as flushing liquid.

14. The process according to one of claims 8 to 12, characterized by the use of a disinfectant as an addition to the flushing liquid.

15. The process according to one of claims 8 to 12, characterized by the use of fluorides as additive to the flushing liquid.

## Revendications

1. Pièce à main (1) pour soins dentaires avec un faisceau laser (8), comportant un boîtier (2), avec une fibre optique laser (3) maintenue dans le boîtier (2), et avec un dispositif de rinçage (4), qui dirige un jet de liquide (6) dans la zone du point de travail du faisceau laser (8), le boîtier (2) entourant coaxialement la fibre optique (3), et le faisceau laser (8) étant couplé coaxialement au jet de liquide (6), de telle sorte que le jet de liquide est utilisable comme fibre optique,
caractérisée par le fait que le boîtier (2) présente un conduit (10) dans lequel le liquide est concentré en un jet de liquide (6) laminaire jusqu'à l'extrémité de sortie du conduit (10), et par le fait que l'extrémité de la fibre optique (3) est disposée à l'intérieur du conduit de boîtier (10) dans le jet de liquide (6) laminaire à distance avant l'extrémité de sortie (12) du conduit de boîtier (10).

2. Pièce à main selon la revendication 1, caractérisée par le fait que l'extrémité (14) de la fibre optique (3) est disposée avant l'extrémité du conduit de boîtier (10) à une distance telle que le cône de lumière laser (16) sortant de la fibre optique (3) parvient au diamètre du jet de liquide (6) à l'extrémité ou immédiatement derrière l'extrémité (12) du conduit de boîtier (10).

3. Pièce à main selon la revendication 1 ou 2, caractérisée par le fait que le débit de liquide délivré s'élève environ à 30 jusqu'à 100 ml/min.

4. Pièce à main selon l'une des revendications 1 à 3, caractérisée par le fait que le diamètre de jet du jet de liquide (6) sortant de l'extrémité du conduit de boîtier (10) avec le faisceau laser (8) couplé s'élève environ à 0,5 jusqu'à 2,5 mm, de préférence environ 1,5 mm.

5. Pièce à main selon l'une des revendications 1 à 4, caractérisée par le fait que le liquide se compose essentiellement d'eau.

6. Pièce à main selon l'une des revendications 1 à 5, caractérisée par le fait que la lumière laser présente une longueur d'onde de 300 à 700 nm, de préférence environ 350 à 520 nm.

7. Pièce à main selon l'une des revendications 1 à 6, caractérisée par le fait que le liquide de rinçage présente un indice de réfraction qui est adapté à l'indice de réfraction de la surface à traiter.

8. Procédé de rinçage du point de travail d'un faisceau laser (8) sortant d'une fibre optique (3), selon lequel un liquide de rinçage est concentré en un jet de liquide (6), le faisceau de lumière laser (8) est couplé dans le jet de liquide (6) concentré, le jet de liquide (6) étant utilisé comme fibre optique (3) et en même temps comme agent de refroidissement pour le point de travail du faisceau laser (8), et un jet de liquide (6) laminaire étant formé dans un conduit de boîtier (10), caractérisé par le couplage coaxial du faisceau laser (8) dans le jet de liquide (6) laminaire à l'intérieur du conduit de boîtier (10) à distance avant l'extrémité de sortie (12) du conduit de boîtier (10).

9. Procédé selon la revendication 8, caractérisé par le fait que le cône de lumière de la lumière laser sortant de la fibre optique (3) est réglé, par le choix d'un diamètre approprié de la fibre optique (3) ou du conduit de boîtier (10), ou par le positionnement correspondant de la fibre optique (14) à l'intérieur du conduit de boîtier (10), de telle sorte que le diamètre du cône de lumière (16) parvienne au diamètre du jet de liquide (6) à l'extrémité (12) du conduit de boîtier (10).

10. Procédé selon l'une des revendications 8 ou 9, caractérisé par le fait que le conduit de boîtier (10), dans le mode contact, est posé sur le point de travail.

11. Procédé selon l'une des revendications 8 ou 9, caractérisé par le fait que le conduit de boîtier (10) est maintenu à distance prédéterminée du point de travail.

12. Procédé selon l'une des revendications 8 à 11, caractérisé par l'utilisation d'un liquide de rinçage dont l'indice de réfraction est adapté à l'indice de réfraction de la surface à traiter.

13. Procédé selon l'une des revendications 8 à 12, caractérisé par l'utilisation d'eau avec une addition de chlorure de calcium comme liquide de rinçage.

14. Procédé selon l'une des revendications 8 à 12, caractérisé par l'utilisation d'un agent désinfectant comme additif au liquide de rinçage.

15. Procédé selon l'une des revendications 8 à 12, caractérisé par l'utilisation de fluorures comme agent d'addition au liquide de rinçage.
